# EUROPEAN PATENT SPECIFICATION

(11) **EP 2 228 026 B1**
(45) Date of publication and mention of the grant of the patent: **06.11.2013**
(21) Application number: 10155206.5
(22) Date of filing: 02.03.2010
(51) Int. Cl.: A61B 17/68, A61B 17/72, A61B 17/56

(54) **A stabilizer device for the mutual elastic stabilization of two bone segments**
Stabilisierungsvorrichtung für die gegenseitige elastische Stabilisierung zweier Knochensegmente
Dispositif stabilisateur pour la stabilisation élastique réciproque de deux segments osseux

(30) Priority: 10.03.2009 IT VR20090026
(43) Date of publication of application: 15.09.2010
(73) Proprietor: Lodola, Giuseppe, 37067 Valeggio sul Mincio (VR) (IT)
(72) Inventor: Lodola, Giuseppe, 37067, Valeggio sul Mincio - Verona (IT); Biz, Carlo, 31029, Vittorio Veneto - Treviso (IT)
(74) Representative: Reniero, Cirillo Silvano

(56) References cited:
- EP-A- 1 952 776
- WO-A-00/06036
- FR-A- 2 878 431
- US-A- 5 807 396
- US-A1- 2003 114 856

## Description

### FIELD OF APPLICATION

The present invention is related to a stabilizer device for fixing two bone segments together in osteotomy operations and in particular in hallux valgus correction operations.

### STATE OF THE ART

As is known, the pathology of hallux valgus is characterized by the progressive lateral deviation of the hallux and by the medial deviation of the first metatarsal. Hallux valgus is the most frequent deformity of the foot and usually is not an isolated anomaly; rather, it represents only the most evident aspect of more complex alterations affecting the entire foot.

Congenital and acquired altered forms exist - the latter especially in women - that are due to mechanical stresses caused by overly pointed footwear and/or footwear with excessively high heel.

Once started, the foot deformity is worsened by the traction of the muscles at the hallux phalanxes. Over time, the base of the first phalanx is progressively externally subluxated and the metatarsal head is moved inwardly. In the soft, overlying parts, a reactive bursitis is formed, which is easily inflamed and causes pain.

The therapeutic treatment of the hallux depends on various parameters which should be carefully considered: patient age, clinical conditions, such as vascular, neurological and metabolic conditions; needs tied to the footwear worn and to the physical activity, and finally the radiological parameters.

There are various treatment methods for this pathology, even if surgical correction is the one which offers the best results, since in most cases the results achieved via surgery are definitive. There are innumerable types of correction surgery operations, including: invasive operations, such as Chevron, Akin or Scarf osteotomy, and mini-invasive operations, such as distal metatarsal osteotomy and proximal osteotomy with the relative balancing of the soft tissues at the distal level, among other operation types.

One operation which is frequently employed today is percutaneous distal osteotomy, which is carried out by means of a small medial incision at the head of the first metatarsal, after which the osteotomy is executed at the base of the neck of the metatarsal head. The translation of the metatarsal head is caused by means of the subungual embedding of a metal wire, passing on the side of the metatarsal head before entering into the metatarsal medullary canal and being distally fixed. The metatarsal head is thus laterally dislocated and maintained due to the action of the metal wire set on its surface and embedded in the metatarsal medullary canal.

This operation type should be considered as a correction aid; the subsequent actual correction will be of triplanar type on physiological basis due to the load of the foot. The metal wire which projects from the patient's skin at subungual level is maintained *in situ* until its removal, 35-40 days after the surgical operation; the same metal wire blocks all distal articulation, in some cases causing a certain articular rigidity in the post-operative time period.

In all of the above-described situations, and in other analogous surgical correction modes of the hallux valgus, several problems have been encountered and described in the scientific literature: some tied to a surgical operation type of invasive type and a rather long post-operative course, others connected with the impossibility of varying the size of the translation between the metatarsal head and the metatarsal segment, still others to the risk of possible frequent correction loss during the immediate post-operative course, to the probable frequent risk of articular rigidity, and to the difficulty of having a quick rehabilitation and hence early healing of the patient. All these difficulties are due to the lack of a suitable stabilizer device between the two bone segments that are the object of osteotomy, which allows selecting during the operation, according to the morphological characteristics of the patient, the translation amount to be carried out between the two affected bone segments. This allows reducing the surgical operation times, which in the post-operative time period prevents articular rigidity and/or the loss of correction, and which ensures the early loading, thus allowing quicker healing. Such elements would benefit the patient, making all the phases of the post-operative rehabilitation period more acceptable.

EP-1 952 776 teaches a stabilizer device including a plate-like element having a slot and a nail component delimiting a longitudinal recess. Moreover, the nail component and the plate-like element are provided with respective knurled portion. The plate-like element and the nail component are connected to one another by screwing a screw into the slot of the plate-like element and the longitudinal recess of the nail component, thereby causing the engagement of the knurled portion of the nail component with the knurled portion of the plate-like component.

The invention is defined in claim 1 while preferred embodiments are set forth in the dependent claims.

The main object of the present invention is to provide a stabilizer device for stabilizing two bone segments, typically after the distal osteotomy of the first metatarsal, in the pathology of hallux valgus. Such device is suitable for carrying out the translation between the two bone segments on the basis of anatomical needs and adjusting the orientation on a frontal and sagittal plane, preventing the mobilization between the two bone segments during bone consolidation and allowing bone joining between the two bone segments, preventing mutual dislocation.

Another object of the present invention is to provide a stabilizer device whose components can be assembled in "reverse" manner, i.e. on one side or the other of a longitudinal axis of the device, so as to allow varying the amount of translation of one bone segment with respect to the other for an optimal realignment of the intermetatarsal angle, thus preventing the mobilization between the two bone segments during bone consolidation without correction loss, and maintaining the two bone segments in the desired position.

Another object of the present invention is to provide a stabilizer device for fixing two bone segments together, after distal osteotomy of the first metatarsal, which allows increasing or decreasing the translation along a frontal plane between the two bone segments, object of osteotomy, thus allowing bone joining without correction losses.

Not the least object of the present invention is to provide a stabilizer device with very simple structure, easy to implant and with competitive manufacturing costs.

These and still other objects, which will be clearer below, are achieved by a stabilizer device for two bone fragments resulting due to axial correction via osteotomy of a metatarsal head with respect to the respective metatarsal segment of the first metatarsal, comprising an endomedullary nail component having a proximal end, a transverse slot and a longitudinal recess made at the head thereof, and a substantially plate-like component, which has a threaded transverse through hole for the engagement of a screw and a shank element projecting overhanging therefrom and configured for the removable engagement with the transverse slot and for removable, yet stable, connection with the longitudinal recess.

Advantageously, the substantially plate-like component is offset with respect to its coupling shank, for this reason, the stabilizer device becomes "reverse" type, as will be further explained below.

The main advantages of a stabilizer device according to the present invention, in addition to the structural simplicity, first of all regard the fact that it uses a device with a plate-like component of "reverse" type, which allows fixing two bone segments together after the distal osteotomy of the first metatarsal in the pathology of hallux valgus, carrying out various translation types between the metatarsal segment of the first digit and the previously osteotomized metatarsal head, preventing the mobilization and the rotation between the two bone segments during bone consolidation, and allowing the bone joining between the two bone segments without correction loss, articular rigidity and infection in the post-operative period.

The plate-like component or plate is meant to be coupled by the operator via engagement with the endomedullary nail component, by exerting an adequate pressure between the plate and the nail components, so as to obtain a stable yet still removable connection between the longitudinal recess and the shank element of the plate. The choice of orientation of the plate in the coupling with the endomedullary nail will be determined by the type of translation that the stabilizer device will have to obtain between a metatarsal bone segment and the respective metatarsal head, i.e. with the median position plane of the plate offset or not offset with respect to the longitudinal axis of the endomedullary nail, also with the possibility to obtain less or more translation, as will be further explained below.

A stabilizer device according to the present invention therefore allows executing a surgical procedure with a mini-invasive technique, with ease and precision, executing the translation type to be carried out between the metatarsal bone segment and the metatarsal head after a distal osteotomy according to surgical needs and as a function of the patient's anatomy, giving maximum stability to the implant in connection with the two involved bone segments, and avoiding possible surgical correction losses, post-operative articular rigidity, rotation between the two bone segments (object of osteotomy), and post-operative infections, up to the attainment of the bone consolidation.

Further characteristics and advantages of the present invention will be clearer from the reading of the following description of its currently preferred embodiments, given as illustrative and non-limiting examples, with reference to the accompanying drawings, in which:
- Figure 1 is a perspective view of a stabilizer device according to a first embodiment of the present invention;
- Figure 2 is a side elevation view of the stabilizer device of Fig. 1;
- Figure 3 is a perspective view according to the present invention with plate-like component in "reverse" position with respect to the stabilizer device of Fig. 1;
- Figure 4 is a side elevation view of the stabilizer device of Fig. 3;
- Figure 5 is a perspective view of a stabilizer device according to the present invention with plate-like component in an intermediate position with respect to the devices of Figures 1 to 4;
- Figure 6 is a side elevation view of the stabilizer device of Fig. 5;

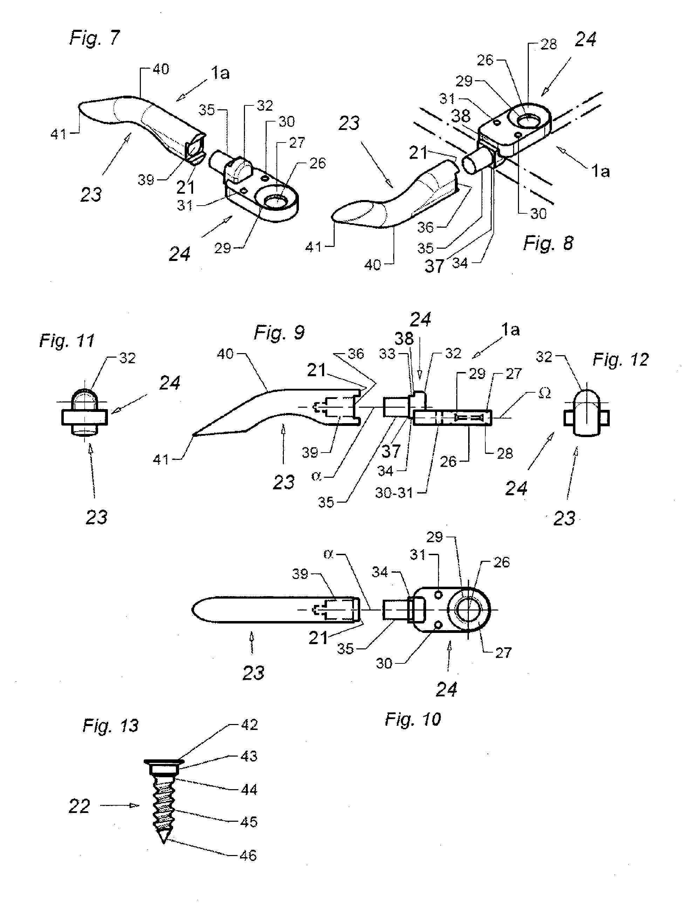

- Figures 7 and 8 are exploded, perspective views of the stabilizer device of Figs. 1 and 2;
- Figures 9, 10, 11 and 12 are, respectively, an exploded elevation view, a plan view, a right side view (with respect to the observer) and a left side view of the stabilizer device of Fig. 7;
- Figure 13 illustrates a two-thread screw;

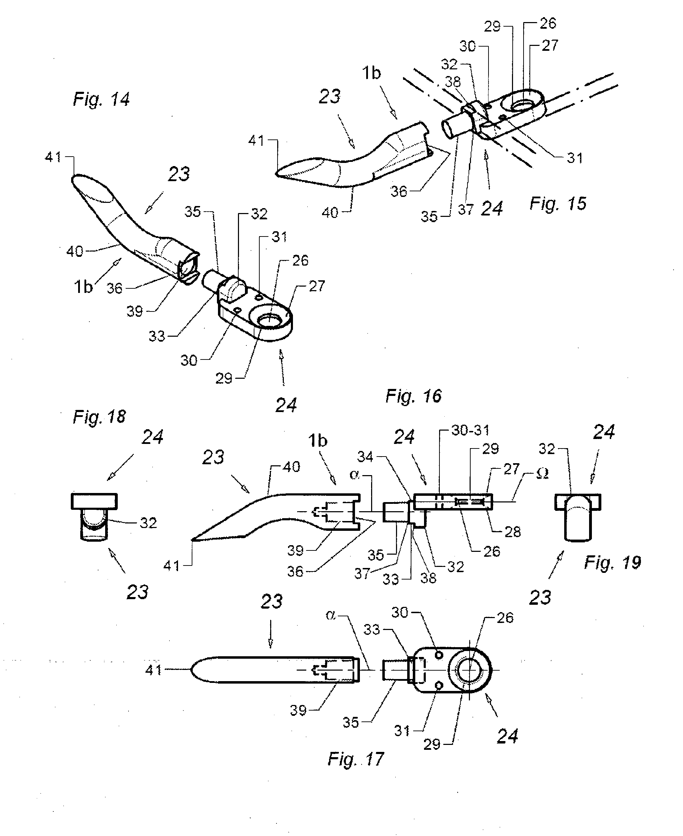

- Figures 14 and 15 are exploded perspective views of the stabilizer device of Figs. 3 and 4;
- Figures 16, 17, 18 and 19 are, respectively, an exploded elevation view, a plan view, a right side view (with respect to the observer) and a left side view of the stabilizer device of Figs. 14 and 15, rotated 180°;
- Figures 20 and 21 are two exploded perspective views of the stabilizer device of Figs. 5 and 6;
- Figures 22, 23, 24 and 25 are, respectively, an exploded elevation view, a plan view, a right side view (with respect to the observer) and a left side view of the stabilizer device of Fig. 20; and
- Figures 26, 27 and 28 illustrate the stabilizer device, respectively, of Figures 1 and 2, 3 and 4, and 5 and 6 implanted between two bone segments, between which it allows relative movement.

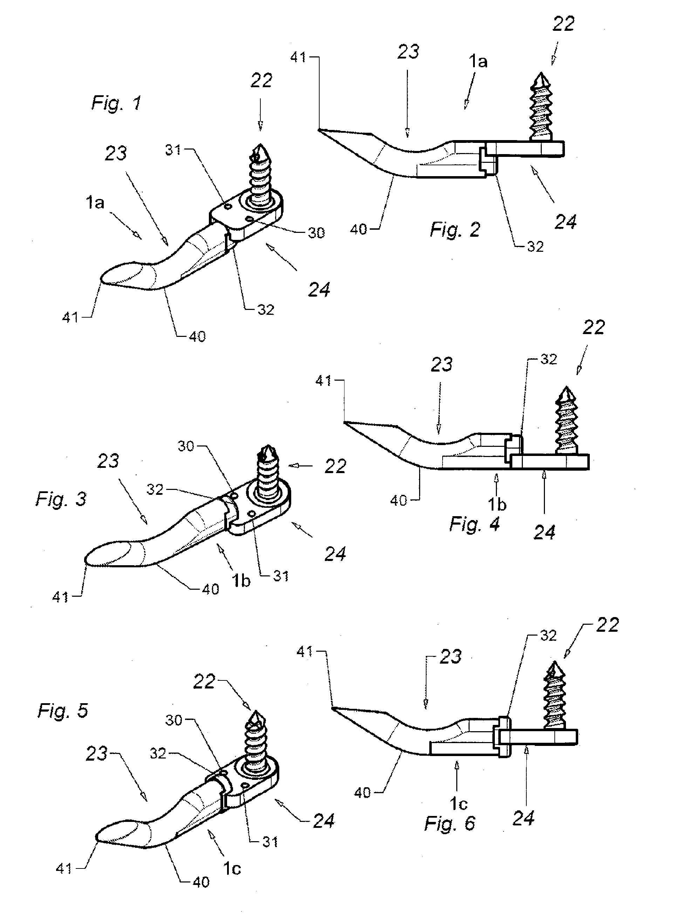

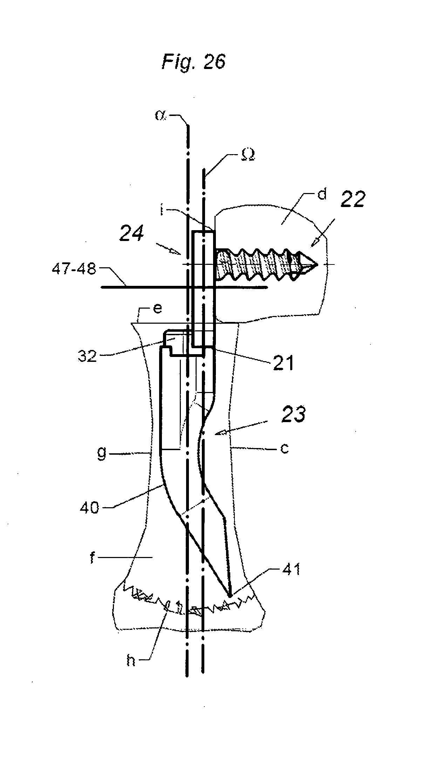

With reference to the above-listed Figures, an embodiment of a stabilizer device (Figs. 1 to 4) is indicated overall with 1a, 1b. Such device is for two adjacent bones or bone fragments resulting after axial correction via osteotomy of a metatarsal head d (Figs. 26 to 28) with respect to the respective metatarsal segment c of the first metatarsal, while with 1c an embodiment is indicated of the same non-"reverse" stabilizer device (Figs. 5 and 6). Each stabilizer device - 1a, 1b and 1c - is formed by an endomedullary nail component 23 and a plate-like component 24.

The nail component 23 has a curved body 40 with a distal end with rounded-off apex 41 and a proximal end 21, at the head thereof a diametrical transverse slot 36 and a longitudinal recess 39 being formed, the latter preferably conical.

The plate-like component 24 has, on one side, a transverse through hole 26 threaded in 29 for engaging a screw 22, preferably with two threads, whereas, on the other side, it has a projecting terminal shank element 35 for the removable coupling with the nail component 23 by means of insertion into the conical recess 39. The terminal shank 35, at its end integral with the plate-like component 24, has a rounded head 32, preferably with circular border, a bit wider than the shank 35 and slightly projecting from the actual plate-like portion 24. In the head 32, a pair of diametrically opposed flat sections 33 and 34 are obtained, such that two abutment shoulders are delimited: one 37 close to the shank 35 and the other 38 between each flat section and the head 32 or the plate-like component 24. The shoulder 37 is designed to abut against the bottom of the slot 36, while the shoulder 38 is meant to abut against the proximal end 21 of the nail component 23.

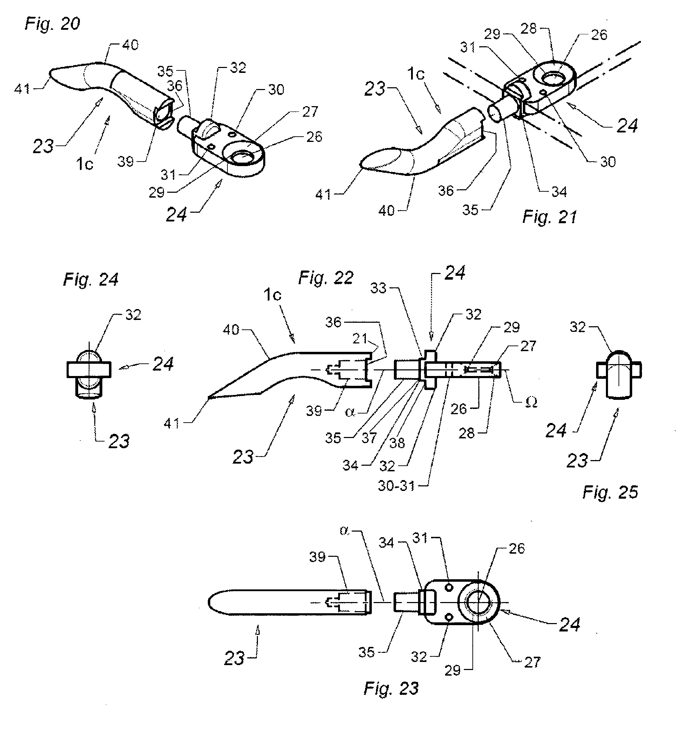

Advantageously, the conical recess 39 has micro-roughness in its surface so as to ensure a good grip on the shank 35 when this is inserted therein. In use, the axis α of the conical recess 39 coincides with the axis of the shank 35 and the intermediate position plane indicated with Ω of the plate-like component 24 is offset, i.e. it does not contain the axis α, in a "reverse" device type, while it contains it in a device of the type illustrated in Figs. 5, 6, 20 to 25 and 28.

The screw 22 preferably has, from one side to the other: a flared head 42, an externally threaded section 43, and a stem equipped with a quick pitch thread 45. Between the externally threaded section 43 and the stem 45, a part without thread 44 can be provided, with slightly smaller diameter than the diameter of the externally threaded section 43 and the diameter of the quick pitch thread 45. The screw also has a pyramidal point 46 in its distal part, which allows improved penetration of the screw 22 in the metatarsal head (d).

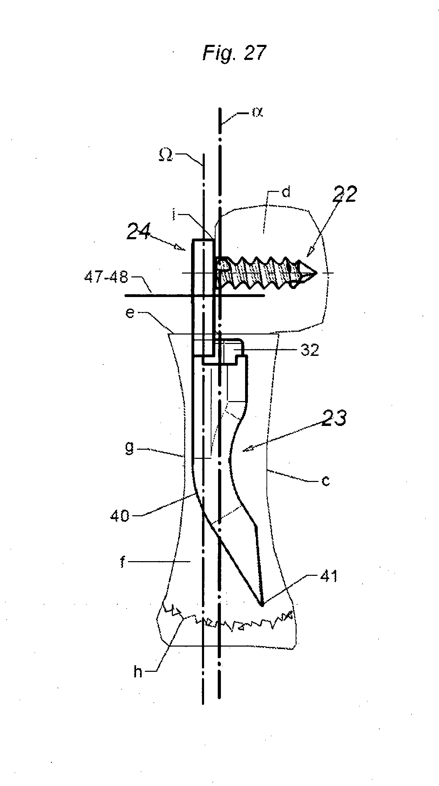

The endomedullary nail 23, as shown in Figures 26 and 27, is designed to be driven into the metatarsal medullary (f) such that its proximal end 21 comes to be situated below the line of the osteotomy (e), its curved part 40 is in abutment on the medial cortical (g) of a metatarsal segment (c) and its rounded-off apex 41 is situated near the base (h) of the medullary canal (f) of the metatarsal bone segment.

The presence of the channel 36 has the function, once the pressure assembly of the two components 23 and 24 has been carried out, of preventing rotation movements between the endomedullary nail 23 component and the plate-like component or plate 24.

Preferably, also the shank 35 of the plate-like component has microroughness on its surface, so as to ensure a good removable engagement between the shank 35 and the recess 39 of the endomedullary nail 23, thus making the endomedullary nail component 23 and the plate 24 integral with each other in use.

The plate-like component or plate 24, once assembled with the endomedullary nail 23, is designed to maintain the translation between the metatarsal head (d) and the metatarsal bone segment (c), resting against the lateral surface (i) of the metatarsal head (d), as shown in Figures 26 and 27.

The hole 26 in the plate 24 is preferably flared, both on the upper face 27 and on the lower face 28, for receiving the screw 22 with flared head 42 from both of its faces (for the "reverse" version). In use, the screw 22 is designed to lock in position the stabilizer device that has been assembled and implanted between the metatarsal bone segment (c) and the metatarsal head (d), as shown in Figures 26, 27 and 28.

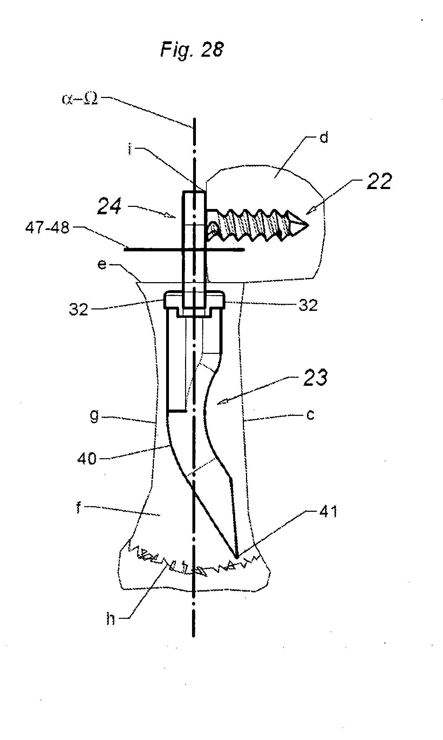

From the procedural standpoint, a pre-operative radiographic investigation is conducted, and the relative translation size to be carried out between the metatarsal bone component (c) and the metatarsal head (d) is determined. The plate-like component or plate 24 is provided for in the most suitable pre-selected position, i.e. if one wishes to carry out a greater translation, one arranges the plate as shown in Fig. 26, or as shown in Fig. 27 if one wishes to carry out a smaller translation, or one uses a non-reverse plate-like component if one wishes to carry out a translation of intermediate type, as shown in Fig. 28. The coupling and stabilization is then carried out between the endomedullary nail 23 and the plate 24 by exerting a specific coupling pressure on them, such that the two components, the nail 23 and the plate 24, will become practically integral with each other.

An osteotomy (e) is then carried out at the base of the neck of the metatarsal head (d). The two components, endomedullary nail 23 and plate 24, once pressure-assembled constitute a monobloc set which is driven into the medullary (f) with the curved part 40 of the endomedullary nail 23 in abutment on the lateral cortical (g) and with the surface of the plate 24 fixed to the lateral surface (i) of the metatarsal head (d). The driving into the medullary (f) is carried out until the proximal part 21 remains below the line of the osteotomy (e). The center of the flared hole 26 of the plate will then be found near the center of the surface (i) of the metatarsal head (d).

The progressive insertion of the assembled stabilizer device into the medullary canal (f) of the metatarsal bone component (c) will cause the gradual translation of the metatarsal head (d) with respect to the metatarsal segment (c) by virtue of the thrust of the surface of the plate 24 in abutment on the surface (i) of the metatarsal head (d) itself, until the previously determined translation size between the two bone components has been attained.

The pre-stabilization of the stabilizer device assembled and inserted in the medullary canal in the above-described manner occurs, as shown in Figures 26 and 27, by means of insertion of two micro-metal wires 47 and 48 (Figs. 26 and 27) through two through (micro)holes 30 and 31 made in the plate-like component 24 in the zone between the hole 26 and the head 32. Such wires, being stuck in the metatarsal head (d), will preserve the temporary stabilization of the assembled device between the metatarsal head (d) and the metatarsal bone segment (c) in order to be able to successively proceed to the radiographic or visual control of the correct translation that occurred and the relative restoration of the intermetatarsal bone angle, between the metatarsal segment (c) and the metatarsal head (d), before proceeding with the definitive fixing by means of locking screw 22, which is screwed into the hole 26 of the plate 24 stuck in the metatarsal head (d) (Figs. 26 and 27). More particularly, the thread 29 of the plate-like component 24 is engaged via screwing with the externally threaded section 43 of the screw 22, whereas the stem equipped with quick pitch thread 45 of the screw is inserted in the metatarsal head (d).

In Figure 28, a "non reverse" stabilizer device is illustrated, implanted and stabilized between the metatarsal segment (c) and the metatarsal head (d), in order to obtain a translation of intermediate type.

## Claims

1. A stabilizer device for two bone fragments resulting after axial correction via osteotomy of a metatarsal head with respect to the respective metatarsal segment of the first metatarsal comprising an endomedullary nail component (23) having a proximal end (21), a transverse slot (36) and a longitudinal recess (39) made at the head thereof, and a substantially plate-like component (24) which has a threaded transverse through hole (26) for the engagement of a screw (22) **characterized in that** it comprises, a shank element (35) projecting overhanging therefrom and configured for the removable engagement with said transverse slot (36) and for removable, yet stable, connection with said longitudinal recess (39).

2. A device according to claim 1, **characterized in that** said endomedullary nail component (23) has a curved body (40) with a distal end with rounded-off apex (41).

3. A device according to claim 1 or 2, **characterized in that** said longitudinal recess (39) is conical.

4. A device according to any preceding claim, **characterized in that** said longitudinal recess (39) has an internal surface with microroughness.

5. A device according to any preceding claim, **characterized in that** said shank element (35) has a head portion (32) wider than said shank (35), in which a pair of diametrically opposed flat sections (33, 34) are made which delimit a pair of shoulders (37, 38) for abutment with said proximal end (21) of said endomedullary nail component (23).

6. A device according to any preceding claim, **characterized in that** said shank element (35) has a substantially frustoconical configuration.

7. A device according to any preceding claim, **characterized in that** said shank element (35) has microroughness on its surface.

8. A device according to any preceding claim, **characterized in that**, in use, the axis (α) of said longitudinal recess (39) is contained in the median position plane (Ω) of said plate-like component (24).

9. A device according to any claim 1 to 6, **characterized in that**, in use, the axis (α) of said longitudinal recess (39) is offset with respect to the median position plane (Ω) of said plate-like component (24), such that the device is of "reverse" type.

10. A device according to claim 9, **characterized in that** said through hole (26) is flared at both ends thereof.

11. A device according to any preceding claim, **characterized in that** said plate-like component (24) has a pair of holes (30, 31) for the insertion therein of a respective working wire (47, 48).

12. A device according to any preceding claim, **characterized in that** said screw (22) has, from one side to the other, a flared head (42), an externally threaded section (43) and a stem equipped with a quick pitch thread (45).

13. A device according to claim 12, **characterized in that** said screw (22) comprises a pyramidal point (46) in its distal part.

## Patentansprüche

1. Eine Stabilisatorvorrichtung für zwei Knochenfragmente, die nach einer axialen mittels Osteotomie vorgenommenen Korrektur eines Köpfchens eines Mittelfußknochens in Bezug auf das jeweilige Mittelfußknochensegment des ersten Mittelfußknochens entstanden sind, aufweisend eine endomedulläre Nagelkomponente (23) mit einem proximalen Ende (21), einem Querschlitz (36) und einer Längsaussparung (39), die an ihrem Kopfende ausgebildet ist, und eine im wesentlichen plattenförmige Komponente (24), die ein mit Gewinde versehenes transversales Durchgangsloch (26) für den Einsatz einer Schraube (22) aufweist, **dadurch gekennzeichnet, dass** sie ein Schaftelement (35) aufweist, das von ihr überhängend vorspringt und zur lösbaren Kupplung mit dem Querschlitz (36) und zur lösbaren, dennoch stabilen Verbindung mit der Längsaussparung (39) konfiguriert ist.

2. Vorrichtung nach Anspruch 1 **dadurch gekennzeichnet, dass** die endomedulläre Nagelkomponente (23) einen gekrümmten Körper (40) mit einem distalen Ende mit abgerundeter Spitze (41) aufweist.

3. Vorrichtung nach Anspruch 1 oder 2 **dadurch gekennzeichnet, dass** die Längsaussparung (39) konisch ist.

4. Vorrichtung nach einem der vorstehenden Ansprüche **dadurch gekennzeichnet, dass** die Längsaussparung (39) eine innere Oberfläche mit Mikrorauhigkeit aufweist.

5. Vorrichtung nach einem der vorstehenden Ansprüche **dadurch gekennzeichnet, dass** das Schaftelement (35) einen breiteren Kopfabschnitt (32) als das Schaftelement (35) aufweist, wobei in dem Kopfabschnitt (32) ein Paar von diametral gegenüberliegenden flachen Abschnitten (33, 34) ausgebildet ist, das ein Paar von Schultern (37, 38) zur Anlage an dem proximalen Ende (21) der endomedullären Nagelkomponente (23) abgrenzt.

6. Vorrichtung nach einem der vorstehenden Ansprüche **dadurch gekennzeichnet, dass** das Schaftelement (35) eine im wesentlichen kegelstumpfförmige Konfiguration aufweist.

7. Vorrichtung nach einem der vorstehenden Ansprüche **dadurch gekennzeichnet, dass** das Schaftelement (35) Mikrorauhigkeit auf seiner Oberfläche aufweist.

8. Vorrichtung nach einem der vorstehenden Ansprüche **dadurch gekennzeichnet, dass** beim Gebrauch die Achse (α) der Längsaussparung (39) in der mittleren Positionsebene (Ω) der plattenförmigen Komponente (24) enthalten ist

9. Vorrichtung nach einem der Ansprüche 1 bis 6 **dadurch gekennzeichnet, dass** beim Gebrauch die Achse (α) der Längsaussparung (39) in Bezug auf die mittlere Positionsebene (Ω) der plattenförmigen Komponente (24) versetzt ist, so dass die Vorrichtung vom "*reversen*" Typ ist.

10. Vorrichtung nach Anspruch 9 **dadurch gekennzeichnet, dass** das Durchgangsloch (26) an seinen beiden Enden konisch erweitert ist.

11. Vorrichtung nach einem der vorstehenden Ansprüche **dadurch gekennzeichnet, dass** die plattenförmige Komponente (24) ein Paar von Löchern (30, 31) jeweils zum Einführen eines Arbeitsdrahts (47, 48) aufweist.

12. Vorrichtung nach einem der vorstehenden Ansprüche **dadurch gekennzeichnet, dass** die Schraube (22) von einer Seite zur anderen einen erweiterten Kopf (42), einen mit einem Außengewinde versehenen Abschnitt (43) und einen Schaft mit einem schnellen Feingewinde (45) aufweist.

13. Vorrichtung nach Anspruch 12 **dadurch gekennzeichnet, dass** die Schraube (22) eine pyramidenförmige Spitze (46) in ihrem distalen Teil aufweist.

## Revendications

1. Un dispositif de stabilisation pour deux fragments d'os obtenus après correction axiale via ostéotomie d'une tête métatarsienne par rapport au segment métatarsien respectif du premier métatarse, comprenant un élément de clou endomédullaire (23) possédant une extrémité proximale (21), une fente transversale (36) et un évidement longitudinal (39) réalisé en tête de celui-ci, et un élément substantiellement en forme de plaque (24) qui possède un trou traversant transversal taraudé (26) pour la venue en prise d'une vis (22),
**caractérisé en ce qu'**il comprend un élément de tige (35) faisant saillie en surplomb et configuré pour la venue en prise amovible avec ladite fente transversale (36) et pour une liaison amovible, quoique stable, avec ledit évidement longitudinal (39).

2. Un dispositif selon la revendication 1, **caractérisé en ce que** ledit élément de clou endomédullaire (23) présente un corps courbé (40) avec une extrémité distale pourvue d'un sommet arrondi (41).

3. Un dispositif selon la revendication 1 ou 2, **caractérisé en ce que** ledit évidement longitudinal (39) est conique.

4. Un dispositif selon l'une des revendications précédentes, **caractérisé en ce que** ledit évidement longitudinal (39) présente une surface interne avec une microrugosité.

5. Un dispositif selon l'une des revendications précédentes, **caractérisé en ce que** ledit élément de tige (35) présente une partie de tête (32) plus large que ladite tige (35), où une paire de parties plates diamétralement opposées (33, 34) sont réalisées qui délimitent une paire d'épaulements (37, 38) pour venue en butée avec ladite extrémité proximale (21) dudit élément de clou endomédullaire (23).

6. Un dispositif selon l'une des revendications précédentes, **caractérisé en ce que** ledit élément de tige (35) présente une configuration substantiellement tronconique.

7. Un dispositif selon l'une des revendications précédentes, **caractérisé en ce que** ledit élément de tige (35) présente une microrugosité à sa surface.

8. Un dispositif selon l'une des revendications précédentes, **caractérisé en ce que**, en utilisation, l'axe (α) dudit évidement longitudinal (39) est contenu dans le plan de position médiane (Ω) dudit composant en forme de plaque (24).

9. Un dispositif selon l'une des revendications 1 à 6, **caractérisé en ce que**, en cours d'utilisation, l'axe (α) dudit évidement longitudinal (39) est décalé par rapport au plan de position médiane (Ω) dudit composant en forme de plaque (24) de sorte que le dispositif soit du type "inverse".

10. Un dispositif selon la revendication 9, **caractérisé en ce que** ledit trou traversant (26) est évasé à ses deux extrémités.

11. Un dispositif selon l'une des revendications précédentes, **caractérisé en ce que** ledit composant en forme de plaque (24) possède une paire de trous (30, 31) pour l'insertion à l'intérieur d'un fil de travail respectif (47, 48).

12. Un dispositif selon l'une des revendications précédentes, **caractérisé en ce que** la vis (22) présente, d'un côté à l'autre, une tête évasée (42), une partie filetée à l'extérieur (43) et une tige pourvue d'un filetage à pas rapide (45).

13. Un dispositif selon la revendication 12, **caractérisé en ce que** ladite vis (22) comprend un point pyramidal (46) à sa partie distale.
